# FASCICULE DE BREVET EUROPEEN

(11) **EP 2 595 568 B1**
(45) Date de publication et mention de la délivrance du brevet: **30.11.2016**
(21) Numéro de dépôt: 11743121.3
(22) Date de dépôt: 13.07.2011
(51) Int. Cl.: A61F 2/18, A61N 1/05, A61B 17/34

(54) **OUTIL D'INSERTION D'OBJETS TUBULAIRES FINS DANS LA COCHLÉE**
WERKZEUG FÜR DEN EINSATZ VON FEINEN TUBUSFÖRMIGEN OBJEKTEN IN DIE OHRSCHNECKE
TOOL FOR INSERTING FINE TUBULAR OBJECTS INTO THE COCHLEA

(30) Priorité: 19.07.2010 FR 1055870
(43) Date de publication de la demande: 29.05.2013
(73) Titulaire: INSERM - Institut National de la Santé et de la Recherche Médicale, 75013 Paris (FR)
(72) Inventeur: BOZORG GRAYELI, Alexis, 75018 Paris (FR); STERKERS, Olivier, 75018 Paris (FR); MIROIR, Mathieu, 75018 Paris (FR); FERRARY, Evelyne, 75890 Paris Cedex 18 (FR); NGUYEN, Yann, 75890 Paris Cedex 18 (FR)
(74) Mandataire: Cabinet Plasseraud
(86) Numéro de dépôt international: PCT/FR2011/051691
(87) Numéro de publication internationale: WO 2012/010783

(56) Documents cités:
- WO-A1-03/070133
- US-A- 6 163 729
- US-A1- 2004 243 177
- US-A1- 2010 114 288
- US-B1- 6 889 094

## Description

La présente invention est relative à un outil d'insertion d'objets tubulaires fins dans la cochlée, tels qu'un implant cochléaire d'une prothèse auditive, ou encore un cathéter.

### ARRIERE-PLAN TECHNOLOGIQUE

On connaît des prothèses auditives comportant un processeur vocal extracranien captant les sons externes pour les convertir en une suite de signaux électriques qui sont envoyés à un émetteur pour être émis en direction d'un récepteur intracranien relié à un implant cochléaire disposé directement dans la cochlée de sorte que celui-ci excite les récepteurs du nerf auditif en réponse à la réception des signaux. L'implant cochléaire a la forme d'un tube fin qui est enfilé dans la cochlée de sorte que le tube s'étende sensiblement dans toute la longueur du parcours curviligne de celle-ci.

L'introduction et la mise en place de l'implant cochléaire dans la cochlée est une opération particulièrement délicate. En effet, la pose de l'implant cochléaire se fait en général sous anesthésie générale, et sous contrôle visuel au-delà du point d'insertion. La façon dont l'implant cochléaire se positionne dans la cochlée est impossible à vérifier extérieurement lors de la pose de l'implant cochléaire, et il faut attendre le scanner de contrôle pour vérifier si l'implant cochléaire a été bien positionné. Si l'implant cochléaire n'est pas bien positionné, il convient alors de retirer celui-ci et d'en poser un autre, ce qui nécessite une nouvelle anesthésie générale.

Pour aider le praticien, il a été développé un outil d'insertion d'objets tubulaires fins dans la cochlée, comportant un corps de préhension terminé par un guide tubulaire dans lequel on place l'implant cochléaire. L'outil comporte un poussoir fin qui coulisse dans le guide tubulaire pour pousser l'implant cochléaire hors du guide afin d'insérer l'implant cochléaire dans la cochlée. Le poussoir est actionné par un index monté coulissant sur le corps de l'outil et qui est poussé par l'un des doigts de la main qui tient l'outil.

Un outil de ce type est décrit dans le document WO 03/070133.

Un outil tel que celui décrit dans le préambule de la revendication 1 est connu du document US-A-2010/0115288. Ce type d'outil comporte plusieurs inconvénients. D'une part, les frottements internes entre l'index et le corps dépassent de loin l'effort résistant d'insertion de l'implant cochléaire dans la cochlée qui est très faible, de sorte qu'il est impossible pour le praticien de ressentir une quelconque résistance inhabituelle lors de ladite insertion, qui pourrait indiquer une insertion problématique. Le praticien pousse donc l'implant cochléaire sans aucune idée de la résistance rencontrée par l'implant cochléaire lors de son insertion. Par ailleurs, le fait que le praticien pousse l'index avec un doigt de la main qui tient l'outil rend le maintien en position de l'outil peu commode, et peut donner lieu à des à-coups lors de l'insertion de l'implant cochléaire.

### OBJET DE L'INVENTION

L'invention a pour objet de proposer un outil d'insertion d'objets tubulaires fins dans la cochlée, ne présentant pas les inconvénients précités.

### BREVE DESCRIPTION DE L'INVENTION

En vue de la réalisation de ce but, on propose un outil d'insertion d'objets tubulaires fins dans la cochlée, comportant un corps de préhension formant une poignée adaptée pour être tenue en main par un utilisateur, ledit corps de préhension étant terminé par un guide tubulaire dans lequel l'objet tubulaire fin est destiné à être disposé, l'outil d'insertion comportant un poussoir engagé dans le guide tubulaire pour pousser l'objet tubulaire fin hors du guide en vue de son insertion dans la cochlée. L'outil d'insertion est équipé de :
- une unité de commande électronique,
- moyens moteurs placés dans le corps de préhension et commandés par l'unité de commande pour déplacer le poussoir,
- moyens de mesure d'effort placés dans le corps de préhension, connectés à l'unité de commande et disposés de sorte à être sensible à un effort de résistance subi par l'objet tubulaire fin lors de son insertion dans la cochlée et fournissant un signal exploitable pour estimer ledit effort résistant,
- moyens d'indication dudit effort résistant placés sur le corps de préhension et connectés à l'unité de commande.

Ainsi, l'outil d'insertion, portable à la main, comprend des moyens moteurs qui peuvent être actionnés automatiquement par l'unité de commande, par exemple par une simple pression sur un interrupteur, ce qui facilite le maintien de l'outil. L'utilisation de moyens moteurs permet une insertion à vitesse sensiblement constante, ce qui élimine tout risque d'à-coup. Enfin, l'indication de l'effort résistant en cours d'insertion permet au praticien de suivre cet effort résistant en temps réel. Cette connaissance permet de détecter plusieurs évènements :
- une résistance finale qui indique que l'objet tubulaire fin est arrivé au bout de la cochlée ; les moyens moteurs peuvent alors être commandés pour arrêter automatiquement la poussée en réponse à la détection d'un seuil d'effort déterminé ;
- une résistance en cours d'insertion qui peut signifier que l'objet tubulaire fin se positionne mal ou que la cochlée est obstruée ; dans ce cas, le praticien, dûment informé de cette résistance, peut choisir d'interrompre l'opération, et éventuellement de la recommencer avec un nouvel objet tubulaire fin, sans attendre le réveil du patient et le scanner de contrôle.

### BREVE DESCRIPTION DES FIGURES

L'invention sera mieux comprise à la lumière de la description qui suit de modes particuliers de réalisation, en référence aux figures des dessins annexés, parmi lesquels :
- la figure 1 est une vue en perspective d'un outil d'aide à l'insertion d'objets tubulaires fins dans la cochlée selon un premier mode particulier de réalisation de l'invention ;
- la figure 2 est une vue en coupe partielle de l'outil de la figure 1 ;
- la figure 3 est une vue en perspective d'un outil d'aide à l'insertion d'objets tubulaires fins dans la cochlée selon un deuxième mode particulier de réalisation de l'invention ;
- la figure 4 est une vue en coupe partielle de l'outil de la figure 3 ;
- la figure 5 est une vue de détail selon la flèche V de la figure 4, le corps ayant été omis ;
- la figure 6 est un graphe de l'effort d'insertion d'un implant cochléaire dans une cochlée en fonction du déplacement du poussoir.

### DESCRIPTION DETAILLEE DE L'INVENTION

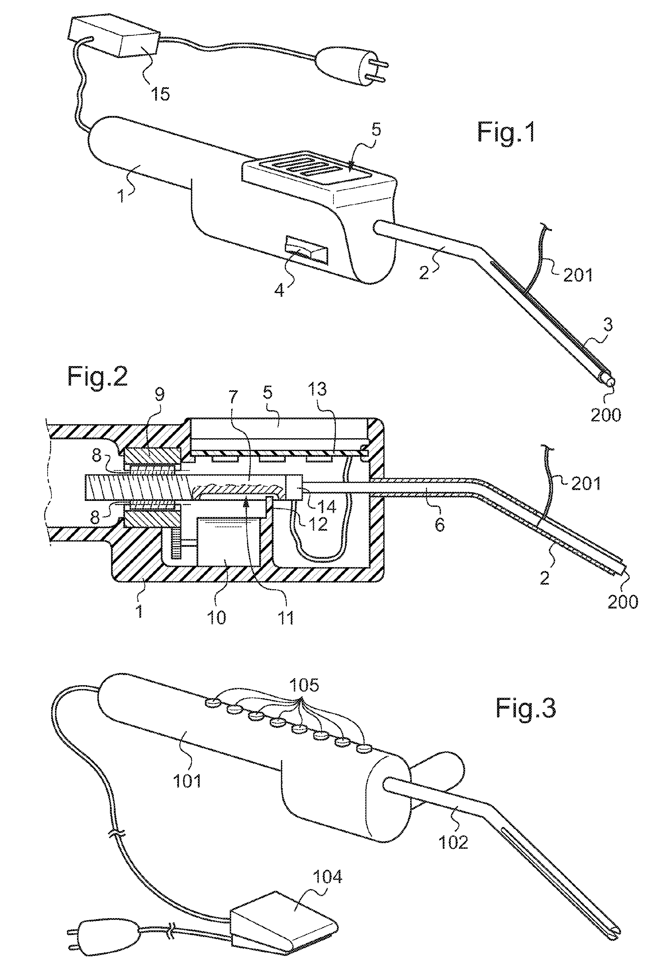

En référence à la figure 1, et conformément à un premier mode particulier de réalisation de l'invention, l'outil d'insertion comporte un corps de préhension 1 formant une poignée qui peut être tenue en main par le praticien ou adapté à l'extrémité d'un robot chirurgical. L'outil d'insertion comporte un guide 2 tubulaire ayant une portion proximale fixée à l'une des extrémités du corps de préhension 1 et une portion distale présentant une extrémité libre et formant ici un coude avec la portion proximale. Le guide 2 reçoit un implant cochléaire 200 dont le fil de liaison 201 sort du guide 2 par une fente longitudinale 3 de ce dernier. Le guide 2 est introduit par le conduit auditif externe après décollement de la peau et du tympan, ou encore par mastoïdectomie et tympanotomie de sorte que son extrémité libre vienne en regard d'un orifice ménagé dans la paroi de la cochlée postérieure (cochléostomie ou fenêtre ronde de la cochlée) pour y introduire l'implant cochléaire 200.

Dans ce mode de réalisation, l'outil d'insertion est équipé d'une unité de commande électronique qui comprend un interrupteur 4 de commande de l'outil d'insertion porté par le corps de préhension, ainsi que d'un écran 5 pour l'information du praticien, et notamment l'indication de l'effort résistant subi par l'implant cochléaire 200 lors de son insertion dans la cochlée, ainsi que cela sera détaillé plus loin.

En référence à la figure 2, l'outil d'insertion comporte un poussoir 6 introduit dans le guide 2 pour pousser l'implant cochléaire 200 à l'intérieur de la cochlée. Au moins la partie terminale du poussoir 6 est flexible pour suivre le coude du guide 2. Le poussoir est lui-même poussé par un piston 7 monté coulissant dans le corps de préhension 1 selon un axe de déplacement coïncidant avec un axe longitudinal de la portion proximale du guide 2. Le piston 7 a une partie filetée qui coopère avec des rouleaux filetés 8 qui coopèrent eux-mêmes avec une couronne 9 qui est montée à rotation sur le corps de préhension 1, à l'intérieur de celui-ci, de sorte qu'une rotation de la couronne 9 entraîne un déplacement longitudinal du piston 7 et, partant, un déplacement longitudinal du poussoir 6.

La rotation de la couronne 9 est provoquée au moyen d'un moteur électrique 10 placé dans le corps de préhension 1 et commandé par l'unité de commande pour entraîner la couronne 9 par l'intermédiaire d'un pignon coopérant avec une denture externe de la couronne 9. Pour empêcher le piston 7 de tourner, celui-ci comporte une rainure longitudinale 11 dans laquelle un doigt 12 solidaire du corps de préhension 1 est engagé.

L'interrupteur 4 de l'unité de commande est un interrupteur à trois positions dont une position intermédiaire stable vers laquelle l'interrupteur est rappelé en l'absence d'appui et dans laquelle le moteur électrique 10 n'est pas alimenté. Dans les deux autres positions, le moteur électrique est alimenté pour tourner dans un sens ou dans l'autre.

L'unité de commande comprend également une carte électronique 13 qui est contenue dans le corps de préhension 1 et qui porte des composants électroniques gérant l'alimentation du moteur électrique 10 ainsi que l'écran 5.

Ici, l'alimentation en électricité est assurée par un transformateur 15 que l'on branche sur le secteur. Le fait de placer le transformateur à l'extérieur du corps de préhension 1 permet d'alléger l'outil, facilitant son maintien lors de l'insertion de l'implant cochléaire.

Selon un aspect essentiel de l'invention, un capteur d'effort 14, ici un capteur de type piézoélectrique, est interposé entre le piston 7 et le poussoir 6 pour mesurer un effort résistant subi par le poussoir 6.

L'effort résistant subi par le poussoir 6 est la somme d'un effort de frottement entre le guide et le poussoir et d'un effort résistant subi par l'implant cochléaire lors de son insertion dans la cochlée. Or l'effort de frottement précité est essentiellement constant (à un transitoire près lors de la mise en route du moteur électrique 10) dans la mesure où le moteur électrique 10 tourne à vitesse constante. Il est facile de calibrer cet effort résistant pour le déduire de l'effort mesuré par le capteur d'effort 14, ce qui permet d'obtenir une estimation réaliste de l'effort résistant subi par l'implant cochléaire lors de son insertion dans la cochlée.

La valeur de cet effort résistant est affichée en permanence par l'écran 5, par exemple sous la forme d'une échelle graphique, afin que le praticien puisse en prendre connaissance en temps réel. Si l'effort résistant monte anormalement en cours d'insertion, c'est certainement que l'implant cochléaire se positionne mal dans la cochlée. Le praticien est ainsi immédiatement informé du problème.

En outre, en fin de course, l'implant cochléaire vient buter contre le fond de la cochlée et l'effort résistant affiché monte franchement. Le praticien est ainsi informé que l'opération est terminée et il peut relâcher l'interrupteur. En variante, la commande du moteur électrique 10 peut être agencée pour arrêter le moteur électrique dès que l'effort résistant atteint un seuil déterminé.

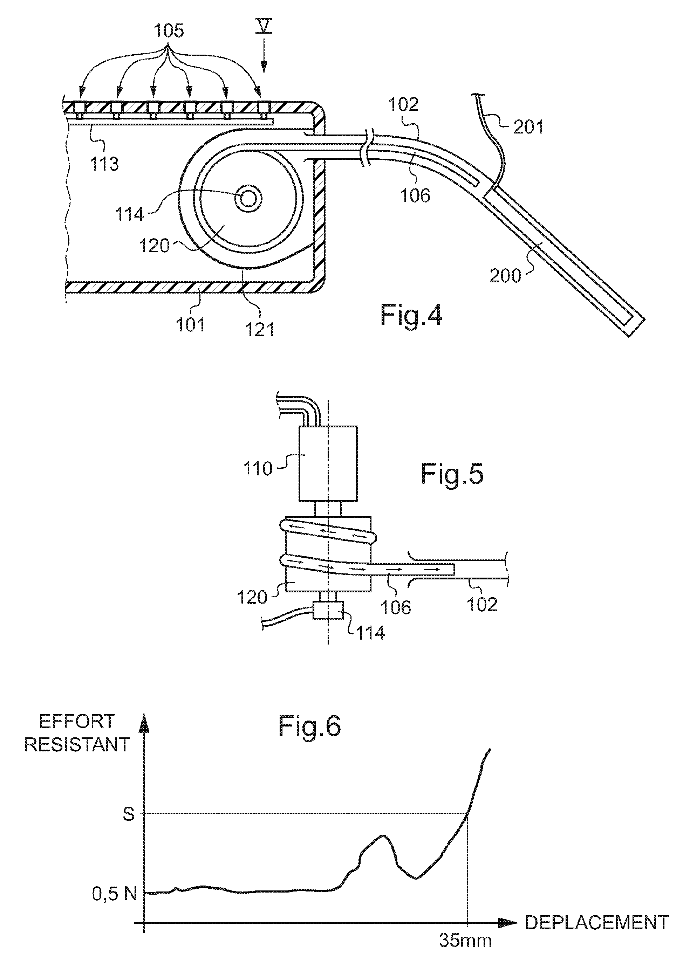

Les inventeurs ont effectué diverses mesures qui ont permis de constater que l'effort résistant subi par l'implant cochléaire lors de son insertion dans la cochlée a la forme générale illustrée par la courbe de la figure 6 sur laquelle les valeurs ne sont données qu'à titre indicatif et peuvent varier d'un patient à l'autre. Cette courbe présente un premier plateau d'effort relativement constant, puis vers la fin de l'insertion, présente un pic transitoire suivi d'un relâchement, avant de monter franchement lorsque l'implant cochléaire atteint le fond de la cochlée. En choisissant un seuil S supérieur au pic transitoire, mais susceptible d'être atteint lors de la montée finale de l'effort résistant, on assure que le moteur est bien arrêté à la fin de l'insertion.

Selon une première variante, le seuil est prédéterminé une fois pour toutes et valable pour tous les patients. Selon une deuxième variante, l'unité de commande intègre un algorithme qui reconnaît en temps réel la forme de la courbe d'effort résistant et repère le pic transitoire suivi du relâchement. Si un tel relâchement ne se produit pas, c'est que l'implant cochléaire est mal positionné. Le moteur est alors immédiatement arrêté lorsque l'effort atteint un seuil prédéterminé, et un message correspondant est affiché sur l'écran 5. Si un tel relâchement se produit, c'est a priori que l'insertion de l'implant cochléaire se passe normalement. L'algorithme calcule alors un seuil d'effort adapté, par exemple égal à 1,5 fois la valeur du pic transitoire, seuil qui servira à arrêter le moteur électrique 10 en fin d'insertion.

Selon maintenant un deuxième mode de réalisation illustré aux figures 3 à 5, et sur lesquelles les références des éléments communs avec les figures 1 et 2 sont augmentées d'une centaine, l'outil d'insertion de l'invention, portable tout comme celui du premier mode de réalisation, comporte un corps de préhension 101 avec un guide 102. Le poussoir 106 a son extrémité distale engagée dans le guide 102, et est enroulé sur un tambour 120 monté tournant sur le corps de préhension 101 selon un axe de rotation transversal, et qui est ici directement entraîné par le moteur électrique 110 s'étendant selon l'axe de rotation du tambour 120. Sur la figure 3, on voit le moteur 110 saillir sur le côté du corps 101, de sorte à former une poignée de préhension facilitant la prise en main de l'outil. Un carter 121 entourant le tambour 120 contraint le poussoir 106 pour le faire entrer dans le guide 102 au fur et à mesure de son déroulement.

Le capteur d'effort 114 est ici un capteur de couple qui mesure le couple résistant subi par le tambour 120 lors de sa rotation. Le capteur de couple 114 mesure un couple proportionnel à la somme de l'effort de frottement que subit le poussoir 106 et de l'effort résistant que subit l'implant cochléaire 200 lors de son insertion dans la cochlée. L'effort de frottement étant sensiblement constant, il suffit de le soustraire de la mesure donnée par le capteur de couple 114 pour obtenir une estimation de l'effort résistant subi par l'implant cochléaire lors de son insertion.

Dans ce mode de réalisation particulier, l'indication de l'effort résistant est effectué au moyen d'une série de LEDs 105 qui s'allument les unes après les autres au fur et à mesure que l'effort résistant subi par l'implant cochléaire lors de son insertion dans la cochlée augmente.

Ici, l'interrupteur du mode de réalisation précédent a été remplacé par une pédale 104 commandant la mise en rotation du moteur. Ainsi les mains du praticien ne sont utilisées que pour maintenir l'outil.

La pédale 104 renferme ici le transformateur 115 dont on voit dépasser le fil d'alimentation terminé par une prise pour le branchement de l'outil sur le secteur. Ainsi, l'outil est très léger et facile à maintenir en place.

L'invention n'est bien entendu pas limitée à ce qui vient d'être décrit, mais englobe au contraire toute variante entrant dans le cadre défini par les revendications.

En particulier, bien que l'on ait indiqué que l'alimentation du moteur et des moyens d'indication était assurée par un transformateur externe, on pourra bien sûr équiper l'outil de piles ou de batteries logées dans le corps de l'outil.

Bien que l'on ait indiqué que les moyens d'indication comportaient un écran ou une série de LEDs, on pourra bien sûr indiquer l'effort résistant subi par l'implant cochléaire lors de son insertion par tout autre moyen, par exemple par un dispositif sonore produisant un son dont l'intensité ou la hauteur dépend de la valeur de l'effort résistant.

Bien que l'on ait indiqué que l'on utilisait l'outil pour insérer un implant cochléaire, on pourra également utiliser l'outil pour insérer tout autre objet tubulaire fin, comme par exemple un cathéter pour délivrer un traitement directement à l'intérieur de la cochlée.

Bien que l'on ait indiqué que le praticien tenait l'outil par le corps de celui-ci, le corps de l'outil pourra être prévu adaptable à l'extrémité d'un robot chirurgical ou sur la platine d'un support fixe maintenant l'outil immobile lors de l'insertion.

Bien que l'on ait indiqué ici que les moyens moteurs comprenaient un moteur électrique, on pourra utiliser d'autres moyens moteurs, comme un moteur pneumatique ou hydraulique.

Bien que l'on ait indiqué que l'outil était équipé de moyens de mesure d'effort sous la forme d'un capteur d'effort inséré dans la chaîne de transmission entre le moteur électrique et le poussoir de façon à être sensible à l'effort résistant subi par l'implant cochléaire lors de son insertion dans la cochlée, on pourra implanter le capteur d'effort à d'autres endroits que ceux qui ont été illustrés, comme par exemple à l'extrémité du poussoir, pour mesurer directement l'effort résistant que subit l'objet poussé par le poussoir. On pourra également utiliser d'autres moyens qu'un capteur d'effort, par exemple des moyens de mesure du courant circulant dans les bobines du moteur afin d'estimer le couple généré par celui-ci.

Enfin, on pourra équiper l'outil d'autres moyens de mesure, comme par exemple un gyroscope pour détecter tout mouvement de la direction nominale de la portion distale du guide lors de l'insertion. De préférence, le gyroscope sera alors couplé à des moyens d'indication pour indiquer au praticien dans quelle direction redresser l'outil afin de retrouver la direction nominale de la portion distale du guide lors de l'insertion.

En outre, on pourra équiper l'outil d'une fibre optique courant le long du guide pour permettre au praticien de voir l'intérieur de l'oreille interne du patient lors de l'opération d'insertion.

## Revendications

1. Outil d'insertion d'objets tubulaires fins dans la cochlée, comportant un corps de préhension (1;101) formant une poignée adaptée pour être tenue en main par un utilisateur, ledit corps de préhension étant terminé par un guide (2;102) tubulaire dans lequel l'objet tubulaire fin est destiné à être disposé, l'outil d'insertion comportant un poussoir (6;106) engagé dans le guide tubulaire pour pousser l'objet tubulaire fin hors du guide en vue de son insertion dans la cochlée,
ledit outil d'insertion étant **caractérisé en ce qu'**il est équipé de :
- une unité de commande électronique,
- moyens moteurs (10;110) placés dans le corps de préhension et commandés par l'unité de commande pour déplacer le poussoir,
- moyens de mesure d'effort (14;114) placés dans le corps de préhension, connectés à l'unité de commande et disposés de sorte à être sensible à un effort de résistance subi par l'objet tubulaire fin lors de son insertion dans la cochlée et fournissant un signal exploitable pour estimer ledit effort résistant,
- moyens d'indication (5;105) dudit effort résistant placés sur le corps de préhension et connectés à l'unité de commande.

2. Outil d'insertion selon la revendication 1, dans lequel les moyens de mesure d'effort comportent un capteur d'effort (14) disposé entre le poussoir introduit dans le guide pour pousser l'objet tubulaire fin et un piston (7) actionné par les moyens moteurs.

3. Outil d'insertion selon la revendication 1, dans lequel les moyens de mesure d'effort comporte un capteur de couple (114) disposé pour mesurer un couple subi par un tambour (120) qui est monté tournant dans le corps pour être entraîné en rotation par les moyens moteurs, et sur lequel le poussoir est enroulé.

4. Outil d'insertion selon la revendication 1, dans lequel les moyens d'indication comportent un écran d'affichage (5) disposé directement sur le corps de l'outil.

5. Outil d'insertion selon la revendication 1, dans lequel les moyens d'indication comportent une série de LEDs (115) disposés directement sur le corps de l'outil et qui s'allument les unes après les autres au fur et à mesure que l'effort résistant subi par l'implant cochléaire lors de son insertion dans la cochlée augmente.

6. Outil d'insertion selon la revendication 1, dans lequel les moyens moteurs sont commandés pour interrompre une poussée du poussoir lorsque l'effort résistant dépasse un seuil (S) donné.

7. Outil selon la revendication 6, dans lequel le seuil (S) est prédéterminé.

8. Outil selon la revendication 6, dans lequel le seuil (S) est fixé en fonction d'une valeur d'un pic transitoire d'effort résistant détecté lors de l'insertion de l'objet tubulaire fin dans la cochlée.

## Patentansprüche

1. Werkzeug zum Einführen von feinen rohrförmigen Objekten in die Cochlea, umfassend einen Greifkörper (1; 101), welcher einen Griff bildet, welcher dazu eingerichtet ist, von einem Benutzer mit der Hand gehalten zu werden, wobei der Greifkörper in einer rohrförmigen Führung (2; 102) endet, in welcher das feine rohrförmige Objekt anzuordnen ist, wobei das Werkzeug zum Einführen einen Stößel (6; 106) umfasst, welcher in der rohrförmigen Führung aufgenommen ist, um das feine rohrförmige Objekt aus der Führung heraus zu schieben, um es in die Cochlea einzuführen,
wobei das Werkzeug zum Einführen **dadurch gekennzeichnet ist, dass** es ausgerüstet ist mit:
- einer elektronischen Steuereinheit,
- Antriebsmitteln (10, 110), welche in dem Greifkörper angeordnet sind und durch die Steuereinheit angesteuert werden, um den Stößel zu verlagern,
- Mitteln zum Messen einer Kraft (14; 144), welche in dem Greifkörper platziert sind, mit der Steuereinheit verbunden sind und derart eingerichtet sind, dass sie eine Widerstandskraft erfassen, welche von dem feinen rohrförmigen Objekt bei seinem Einführen in die Cochlea erfahren wird, und ein Signal liefern, welches verwendbar ist, um die Widerstandskraft abzuschätzen,
- Mitteln zum Anzeigen (5; 105) der Widerstandskraft, welche an dem Greifkörper platziert und mit der Steuereinheit verbunden sind.

2. Werkzeug zum Einführen nach Anspruch 1, wobei die Mittel zum Messen einer Kraft einen Kraftsensor (14) umfassen, welcher zwischen dem Stößel, welcher in der Führung zum Schieben des feinen rohrförmigen Objekts aufgenommen ist, und einem Kolben (7) angeordnet ist, welcher durch die Antriebsmittel betätigt wird.

3. Werkzeug zum Einführen nach Anspruch 1, wobei die Mittel zum Messen einer Kraft einen Drehmomentsensor (114) umfassen, welcher dazu angeordnet ist, ein von einer Trommel (120) erfahrenes Drehmoment zu messen, welche drehbar in dem Körper montiert ist, um von den Antriebsmitteln in Rotation versetzt zu werden, und auf welche der Stößel aufgewickelt ist.

4. Werkzeug zum Einführen nach Anspruch 1, wobei die Anzeigemittel einen Bildschirm (5) umfassen, welcher direkt an dem Werkzeugkörper angeordnet ist.

5. Werkzeug zum Einführen nach Anspruch 1, wobei die Anzeigemittel eine Reihe von LEDs (115) umfassen, welche direkt an dem Werkzeugkörper angeordnet sind und eine nach der anderen aufleuchten, wenn die von dem Cochlea-Implantat erfahrene Widerstandskraft während seines Einführens in die Cochlea zunimmt.

6. Werkzeug zum Einführen nach Anspruch 1, wobei die Antriebsmittel angesteuert werden, um einen Vorschub des Stößels zu unterbrechen, wenn die Widerstandskraft einen gegebenen Schwellenwert (S) übersteigt.

7. Werkzeug nach Anspruch 6, wobei der Schwellenwert (S) vorbestimmt ist.

8. Werkzeug nach Anspruch 6, wobei der Schwellenwert (S) gemäß einem Wert eines vorübergehenden Spitzenwerts der Widerstandskraft festgelegt wird, der während des Einführens des feinen rohrförmigen Objekts in die Cochlea detektiert wird.

## Claims

1. Tool for insertion of thin tubular objects in the cochlea, comprising a gripping tool (1;101) forming a handle adapted to be held in a user's hand, said gripping body being terminated by a tubular guide (2,102) in which the thin tubular object will be placed, said insertion tool comprising a pusher (6;106) engaged in the tubular guide to push the thin tubular object outside the guide so that it can be inserted in the cochlea,
said insertion tool being **characterised in that** it is fitted with:
- an electronic control unit
- driving means (10;110) placed in the gripping body and controlled by the control unit to move the pusher,
- force measurement means (14,114) placed in the gripping body, connected to the control unit and arranged so as to be sensitive to a resistive force applied on the thin tubular object as it is being inserted into the cochlea and outputting a signal that can be used to estimate said resistive force,
- means (5;105) of indicating said resistive force placed on the gripping body and connected to the control unit.

2. Insertion tool according to claim 1, in which the force measurement means comprise a force sensor (14) located between the pusher inserted into the guide to push the thin tubular object and a piston (7) actuated by the driving means.

3. Insertion tool according to claim 1, in which the force measurement means comprise a torque sensor (114) arranged to measure a torque applied to a drum (120) that is installed free to rotate in the body to be driven in rotation by the driving means and on which the pusher is wound.

4. Insertion tool according to claim 1, in which the indication means comprise a display screen (5) located directly on the body of the tool.

5. Insertion tool according to claim 1, in which the indication means comprise a series of LEDs (115) located directly on the body of the tool and that light up one after the other as the resistive force applied to the cochlear implant increases as it is inserted into the cochlea.

6. Insertion tool according to claim 1, in which the driving means are controlled to interrupt the thrust of the pusher when the resistive force exceeds a given threshold (S).

7. Tool according to claim 6, in which the threshold (S) is predetermined.

8. Tool according to claim 6, in which the threshold (S) is fixed as a function of the value of a transient peak resistive force detected during insertion of the thin tubular object into the cochlea.
